# EUROPEAN PATENT SPECIFICATION

(11) **EP 1 811 939 B1**
(45) Date of publication and mention of the grant of the patent: **09.03.2011**
(21) Application number: 05798461.9
(22) Date of filing: 04.10.2005
(51) Int. Cl.: A61Q 15/00, A61K 8/04, A61K 8/06, A61K 8/26, A61K 8/28, A61K 8/29, A61K 8/34, A61K 8/38, A61K 8/81, A61K 8/891, A61K 8/898, A45D 34/00, B65D 83/14

(54) **ANTIPERSPIRANT AEROSOL PRODUCT WITH PACKAGING TREATED AGAINST CORROSION**
ANTIPERSPIRANS-AEROSOL MIT GEGEN KORROSION BEHANDELTER VERPACKUNG
PRODUIT AEROSOL ANTISUDORAL DONT LE CONDITIONNEMENT EST TRAITE CONTRE LA CORROSION

(30) Priority: 13.11.2004 EP 04257056
(43) Date of publication of application: 01.08.2007
(73) Proprietor: Unilever PLC, London EC4Y 0DY (GB); Unilever N.V., 3013 AL Rotterdam (NL)
(72) Inventor: FORBES, Karen Elizabeth, Seacrof Yorkshire LS14 2AR (GB); FRANKLIN, Kevin R., Wirral Merseyside CH63 3JW (GB); SHEARMUR, Thomas E., Merseyside CH63 3JW (GB)
(74) Representative: Whaley, Christopher
(86) International application number: PCT/EP2005/010873
(87) International publication number: WO 2006/050776

(56) References cited:
- EP-A- 0 812 182
- WO-A-2004/019862
- WO-A-2004/064833
- CH-A- 557 175
- DE-A1- 10 155 792
- US-A- 6 045 784

## Description

### Field of Invention

This invention is concerned with antiperspirant aerosol products. In particular, this invention is concerned with the composition and packaging of antiperspirant aerosol products.

### Background

Many types of antiperspirant aerosol product exist. Most are packaged in metal cans capable of withstanding the pressure exerted by a volatile propellant that is generally a component of such systems. The composition of such products is typically an anhydrous suspension of particulate antiperspirant active in an oily continuous phase. Unfortunately, such compositions can lead to the problem of valve blockage of the dispensing device used with the composition, because of the particulate nature of the antiperspirant active that they contain. There can also be a problem with "settling out" of the antiperspirant active on storage of the packaged product, this necessitating the shaking of the product prior to each application in order to re-disperse the active.

The above problems may be overcome by using a composition in which the antiperspirant active is dissolved in water. Unfortunately, the presence of water in an aerosol product introduces the problem of corrosion of the metal can from which the composition is typically dispensed.

Numerous compositions in which the antiperspirant active is dissolved in water are disclosed in the art. Some of these disclose water-in-oil emulsion compositions in which an aqueous solution of antiperspirant active is emulsified in an oily continuous phase.

US 4,695,451 (Straw et al) discloses an antiperspirant aerosol composition in the form of a substantially stable water-in-oil emulsion.

EP 812,182 B1 (Correia) discloses an antiperspirant aerosol composition comprising a base in the form of a water-in-oil emulsion stabilised by a silicone surfactant and packaged in an aluminium can.

The article "New Approaches to Antiperspirant and deodorant Formulation" by A. J. Disapio in HAPPI, February 1986, discloses the use of silicone oils in water-in-oil antiperspirant aerosol emulsion compositions.

WO 94/22420 (Shaw) discloses silicone-based water-in-oil micro-emulsions that employ a silicone emulsifying agent and various polyols that could theoretically act as emollients.

EP 373,424 B1 (Raleigh et al) discloses an antiperspirant composition in the form of a water-in-oil emulsion stabilised by a silicone surfactant and an organic surfactant.

US 4,264,586 (Callingham et al) discloses an antiperspirant composition suitable for spray application that is in the form of a water-in-oil emulsion comprising a wax dissolved in polydimethylsiloxane.

US 4,268,499 (Keil) discloses an antiperspirant composition in the form of a water-in-oil emulsion having methylsiloxane fluid as the continuous phase and stabilised by a three-component emulsifying system.

### Summary of Invention

We have discovered that the problems referred to above may be solved by the use of an antiperspirant aerosol product in which the antiperspirant active is dissolved in water, the resulting aqueous solution being emulsified in an oily continuous phase, and the oil-in-water emulsion composition being packaged in a dispensing device comprising a particular selection of lacquered components.

Thus, in a first aspect of the invention, there is provided an antiperspirant aerosol product comprising an aqueous solution of antiperspirant active emulsified in an oily continuous phase and packaged in a dispensing device comprising an aluminium can body internally lacquered with PAM and a mounting cup lacquered on its internal surface with EP-vinyl lacquer.

In a second aspect of the invention, there is provided a method of dispensing an antiperspirant aerosol composition comprising an aqueous solution of antiperspirant active emulsified in an oily continuous phase, said method comprising:
i. the containment of said antiperspirant aerosol composition in a pressurised state in a dispensing device comprising an aluminium can body internally lacquered with PAM and closed by a mounting cup lacquered on its internal surface with EP-vinyl lacquer ;
ii. releasing said pressurised antiperspirant aerosol composition by the opening a valve located in the middle of the mounting cup; and
iii. passing the antiperspirant aerosol composition through a nozzle to produce a spray.

In a third aspect of the invention, there is provided a method of manufacture of an antiperspirant aerosol product comprising the steps of:
i. dissolving an antiperspirant active in water;
ii. emulsifying the resulting aqueous solution of antiperspirant active in an oily continuous phase;
iii. packaging of resulting the oil-in-water emulsion composition in a dispensing device comprising an aluminium can body internally lacquered with PAM and a mounting cup lacquered on its internal surface with EP-vinyl lacquer.

The present invention provides an antiperspirant aerosol product having good dispensing properties and stability. The product is particularly stable with respect to corrosion resistance. The good dispensing properties arise because the antiperspirant composition comprised within the product does not contain any particulate antiperspirant active, the antiperspirant active being dissolved in an aqueous phase.

The aqueous solution of antiperspirant active used in the current invention is typically acidic and corrosive in nature. Despite this fact, good corrosion resistance is provided by the present invention. This is due to the emulsification of the aqueous phase in an oily continuous phase, the selection of aluminium as the material for the can body, the selection of PAM as a lacquer for the internal surface of the aluminium can body, and the selection of EP-vinyl lacquer as a lacquer for the internal surface of the mounting cup.

The presence of water in the products of the invention can lead to them being more environmentally acceptable, as the water may replace a VOC (volatile organic carbon) component. The presence of VOCs in the atmosphere has been linked with various health and environmental problems and there is an increasing desire to minimise the VOC content of consumer products. This is a further objective of the present invention.

### Detailed Description

The antiperspirant aerosol product according to the invention comprises an antiperspirant aerosol composition comprising an aqueous solution of antiperspirant active emulsified in an oily continuous phase. Other components may optionally be present in the composition.

The antiperspirant active is typically selected from astringent salts including, in particular, aluminium, zirconium, and mixed aluminium-zirconium salts, including both inorganic salts, salts with organic anions, and complexes. Preferred antiperspirant actives are aluminium, zirconium, and aluminium-zirconium chlorides, oxychlorides, and chlorohydrates salts. Particularly preferred antiperspirant actives are polynuclear in nature, meaning that the cations of the salt are associated into groups comprising more than one metal ion.

Aluminium halohydrates are especially preferred antiperspirant actives and may be defined by the general formula Al₂(OH)ₓQ_{y}.wH₂O, in which Q represents chlorine, bromine or iodine, x is variable from 2 to 5 and x + y = 6 while wH₂O represents a variable amount of hydration. Aluminium chlorohydrate (ACH) is the most preferred active.

Zirconium salts are usually defined by the general formula ZrO(OH)₂₋ₓQₓ.wH₂O in which Q represents chlorine, bromine or iodine; x is from about 1 to 2; w is from about 1 to 7; and x and w may both have non-integer values. Particular zirconium salts are zirconyl oxyhalides, zirconiun hydroxyhalides, and combinations thereof. Non-limiting examples of zirconium salts and processes for making them are described in Belgian Patent 825,146, Schmitz, issued August 4, 1975 and U.S. Patent 4,223,010 (Rubino).

Antiperspirant actives as used in the invention may be present as mixtures or complexes. Suitable aluminium-zirconium complexes often comprise a compound with a carboxylate group, for example an amino acid. Examples of suitable amino acids include tryptophan, β-phenylalanine, valine, methionine, β-alanine and, most preferably, glycine.

In some embodiments, it is desirable to employ complexes of a combination of aluminium halohydrates and zirconium chlorohydrates with amino acids such as glycine, which are disclosed in US 3,792,068 (Procter and Gamble Co.). Certain of these Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine.

Other actives that may be utilised include astringent titanium salts, for example those described in GB 2,299,506.

Antiperspirant actives are preferably incorporated into compositions of the invention in an amount of from 0.5 to 60%, particularly from 5 to 30% or 40% and especially from 5 or 10% to 30 or 35% by weight.

The antiperspirant active is generally dissolved in water prior to emulsification. In the aqueous antiperspirant active solution that is emulsified, i.e. the disperse phase, the antiperspirant active is preferably present at a concentration of from 10% to 70%, more preferably from 25% to 60%, and most preferably from 30% to 50% by weight of disperse phase. These relatively high concentrations help to minimise the water content without compromising product efficacy. In addition, the amount of water relative to the oily continuous phase is also minimised and corrosion resistance of products according to the invention is thereby enhanced.

The proportion of disperse phase is preferably from 50% to 90%, more preferably from 60% to 80%, and most preferably from 65% to 75% by weight of the composition, excluding any volatile propellant that may be present. These relatively high levels of disperse phase help to minimise the VOC content of the composition (*vide supra) .*

The droplets of aqueous phase dispersed in the oily continuous phase preferably have a relative small particle size, at least 90% of the droplets being from 1 to 25 µm, more preferably from 1 to 12 µm. Particle size determination may be performed using optical microscopy and appropriate image analysis techniques.

The oily continuous phase typically comprises a silicone oil, a hydrocarbon oil, an ester oil, or any mixture thereof. When more than one oil is present, it is highly preferred that the oils are miscible for reasons of phase stability. It is preferred that compositions of the invention comprise a silicone oil in the oily continuous phase. Silicone oils may be cyclic or linear, examples include Dow Corning silicone fluids 344, 345, 244, 245, 246, 556, 704, and the 200 series; Union Carbide Corporation Silicones 7207 and 7158; and General Electric silicone SF1202. Alternatively or additionally, non-silicone oils may be used; such materials include mineral oils, hydrogenated polyisobutene, polydecene, paraffins, isoparaffins of at least 10 carbon atoms, liquid fatty alcohols (e.g. isosteayl alcohol, hexyl decanol, or octyl dodecanol), and aliphatic or aromatic ester oils (e.g. isopropyl myristate, lauryl myristate, isopropyl palmitate, diisopropyl sebecate, diisopropyl adipate, or C₈ to C₁₈ alkyl benzoates.

The oily continuous phase preferably comprises from 10% to 50%, more preferably from 20% to 40%, and most preferably from 25% to 35% by weight of the composition excluding any volatile propellant that may be present.

The oily continuous phase may comprise an emollient oil as one of its component oils. Suitable emollient oils are disclosed in US Patent Nos. 4,822,596 and 4,904,463. Preferred emollient oils are alkyl esters, such as PureSyn Ester 2E7 (neopentylglycol dihexanoate), ex Exxon-Mobil; benzoate esters such as Finsolv TN (Trade Mark), ex Finetex Inc.; hydrogenated polyalkenes, such as Panalane, ex Amoco and PureSyn PAO 2 (hydrogenated polydecene), ex Exxon-Mobil; PPG ethers, such as Fluid AP (PPG-14 butylether), ex Union Carbide; isopropyl palmitate; phenylsilicone; and isopropyl myristate.

An emulsifier is generally required to stabilise the emulsion. The emulsifier has an amphiphilic molecular structure and may be an anionic, cationic, zwitterionic, or nonionic surfactant. An emulsifier that is a nonionic surfactant is preferred. The proportion of emulsifier may be from 0.05% to 5%, preferably from 0.1% to 2.5%, and more preferably from 0.15% to 0.5% by weight of the composition. The preferred levels indicated are the best for phase stability of the emulsion products.

It is desirable to use an emulsifier or a mixture of emulsifiers with an overall HLB value in a range from 2 to 10, preferably from 3 to 8. A mixture of emulsifiers may comprise a surfactant of high HLB and a surfactant of low HLB, blended to give a suitable overall HLB.

High HLB emulsifiers include nonionic esters or ethers comprising a polyoxyalkylene moiety, especially a polyoxyethylene (POE) moiety containing from 2 to 80, and especially from 5 to 60, ethylene oxide (EO) units. Polyoxypropylene (POP) emulsifiers may also be employed, as may emulsifiers comprising one or more polyhydroxylated units such as glycerol, sorbitol, or some other alditol. The emulsifier must also comprise a hydrophobic moiety, for example an alkyl, alkenyl, or aralkyl group, normally containing from about 8 to 50 carbons and particularly from 10 to 30 carbons. The hydrophobic moiety can be either linear or branched and is often saturated, though it can be unsaturated, and it is optionally fluorinated. The hydrophobic moiety can comprise a mixture of chain lengths, for example those deriving from tallow, lard, palm oil, sunflower seed oil or soya bean oil. Examples of suitable high HLB emulsifiers include C₁₆ to C₁₈ alcohols ethoxylated with 10 to 25 ethylene oxide residues and PEG-15-25 stearate or distearate. Other suitable examples include C₁₀-C₂₀ fatty acid mono, di or tri-glycerides. Further examples include C₁₈-C₂₂ fatty alcohol ethers of polyethylene oxides with 8 to 12 EO units.

Low HLB emulsifiers, typically of HLB from 2 to 6, include fatty acid mono- or possibly di-esters of polyhydric alcohols such as glycerol, sorbitol, erythritol or trimethylolpropane. The fatty acyl moiety is often from C₁₄ to C₂₂ and is saturated in many instances, including cetyl, stearyl, arachidyl and behenyl. Examples include monoglycerides of palmitic or stearic acid, sorbitol mono or diesters of myristic, palmitic or stearic acid, and trimethylolpropane monoesters of stearic acid.

Emulsifiers that are silicone derivatives, by which it is meant emulsifiers that have a lipophilic silicone chain, are particularly preferred, especially when the oily continuous phase of the composition comprises a silicone oil. Examples of such emulsifiers include polyoxyalkylene derivatives of dimethylpolysiloxanes, in particular POE, POP, or POE-co-POP derivatives. Such derivatives may terminate in C₁ to C₁₂ alkyl groups. Such emulsifiers may also be dimethicone copolyol silicone surfactants, for example cetyl dimethicone copolyol (sold as Abil EM90, ex Goldschmidt) or lauryl dimethicone copolyol (sold as DC 5200, ex Dow Corning). Emulsifiers that are silicone derivatives are preferably used at from 0.1 to 5%, more preferably 0.15 to 2.5%, and most preferably at from 0.25 to 0.4% by weight of the composition. The preferred levels of emulsifier indicated are the best for phase stability of the emulsion.

In most embodiments of the invention, the product requires the presence of a volatile propellant in the antiperspirant aerosol composition. A volatile propellant serves to pressurise the composition within the dispensing device and to enhance spray formation when the composition is released from the dispensing device via a spray nozzle. When a volatile propellant is present the benefit of the invention is particularly relevant - corrosion resistance being particularly important in a pressurised system.

Suitable volatile propellants include trichlorofluoromethane, trichlorotrifluoromethane, difluoroethane, dimethylether, propane, butane or isobutane or combinations thereof. Preferred volatile propellants are gases at standard temperature and pressure, but are present in the composition in a liquefied state prior to release from the dispensing device. When used, the amount of volatile propellant in the composition of the invention may be from 5 to 95% and is preferably from 30 to 90% by weight of the composition. In order to minimise the VOC content (*vide supra*), the volatile propellant is more preferably from 30 to 65% and most preferably from 30 to 50% by weight of the total composition.

It is advantageous to use volatile propellant at a particular ratio to the water present within the composition in order to have reasonable low VOC content (vide supra) without compromising the corrosion resistance of the product. For this reason, it is preferred that the weight ratio of volatile propellant to water is from 1.5:1 to 5:1; in particular from 1.5:1 to 2.75:1, and especially from 1.5:1 to 2.25:1.

A hydrophobically-modified particulate silica is an optional component of the antiperspirant aerosol composition. Such materials can improve the sensory characteristics of the aerosol product. Suitable silicas are surface-modified to increase their hydrophobicity. Partially hydrophobed silicas are preferred, in particular partially-silylated silicas, and especially partially-silylated silicas having a degree of silylation of from 25% to 75%. The degree of silylation represents the number of silanol (Si-OH) groups of the silica that have been modified by the hydrophobing treatment. Preferred hydrophobically-modified silicas are based on fumed silica. Preferred hydrophobically-modified are organo-silylated. When employed, the hydrophobically-modified particulate silica is typically used at from 0.1% to 3%, in particular at from 0.15% to 1%, and especially at from 0.2% to 0.7% by weight of the total composition.

In certain preferred embodiments of the invention a polymeric co-gellant for the antiperspirant active is employed, as described in WO 02/49590 (Smith et al) and WO 03/105795 (Brown et al). Such polymeric co-gellants comprise Brønsted acid groups and act as co-gellants for the antiperspirant active when mixed therewith in the presence of water, for example water in human sweat, at a temperature of 37°C or less. The polymeric co-gellant may be present as a particulate solid suspended in the oily continuous phase or, preferably, as an aqueous solution emulsified as a separate dispersed phase. When present as an aqueous solution emulsified as a separate disperse phase, it is preferably used as a solution of concentration from 5% to 50%, more preferably from 10% to 30%, and most preferably from 15% to 20% by weight.

When used, the polymeric co-gellant is preferably incorporated into the composition in an amount of from 0.05% to 10%, more preferably from 0.2% to 5% by weight, and most preferably from 1% to 4% by weight of said composition, excluding any volatile propellant present.

Other minor ingredients which may be present in the composition include:
- cosmetically acceptable carrier fluid components, such as straight and branched chain alcohols, for example, ethanol, isobutanol or isopropanol;
- deodorant active perfumes and deodorant compounds which can act as antimicrobial agents;
- inorganic electrolytes, such as sodium chloride or sodium sulphate;
- other rheology modifiers, such as hydroxypropyl celluloses or Bentone 38V;
- a silicone gum, such as Q2 1501, ex Dow Corning;
- polar additives such as propylene carbonate;
- additional skin feel improvers, such as talc and finely divided polyethylene such as Accumist B18;
- humectants, such as polyols, for example glycerol;
- perfumes;
- preservatives and antioxidants;
- skin benefit agents such as allantoin;
- colourants;
- other cosmetic adjuncts conventionally employed in propellant driven aerosol products.

The composition may be manufactured by a method comprising the emulsification of an aqueous solution of antiperspirant active in an oily continuous phase using an emulsifier. Typically the aqueous solution of antiperspirant active is added to the oily continuous phase with stirring.

When a polymeric co-gellant is employed, the composition is preferably prepared as two separate emulsions, one comprising a water-in-oil emulsion of an aqueous solution of the antiperspirant active and the other comprising a water-in-oil emulsion of an aqueous solution of the polymeric co-gellant, the two emulsions being mixed to give the final product as a 'dual emulsion'.

The antiperspirant aerosol product according to the invention comprises a dispensing device comprising an aluminium can body internally lacquered with PAM and a mounting cup lacquered on its bottom side with EP-vinyl lacquer.

It should be noted that numerous lacquers are available for the protection of aluminium can bodies and mounting cups. Examples of such lacquers include epoxyphenolic resin (also called epon-phenolic and often abbreviated to "epoxy" or "EPON"); poly(ethylene terephthalate) (PET); polypropylene; EP-vinyl lacquer (also called organosol or Micoflex); and polyamide imide resin (also called PAM or PAI). The epoxy-phenolic resin is most commonly used.

The aluminium can may be lacquered on its outside; however, it is its internal surface that is in contact with the composition and it is this surface that must be protected by a PAM lacquer. The 'PAM lacquer' is a layer of polyamide imide resin applied to the internal surface of the can by methods known in the art. The layer is preferably of thickness from 1 to 50 microns at all points on the internal surface of the can.

An aluminium can provided with a PAM lacquer on its internal surface has been found to be particularly beneficial in terms of corrosion resistance, when combined with the other features of the present invention (vide infra). For this reason, PAM is the lacquer of choice for the internal surface of the aluminium can, despite the fact this lacquer is particularly difficult to apply.

The mounting cup used with the present invention serves to close off the aluminium can body at its top, typically at a narrowed or 'necked' section of the body. The internal surface of the mounting cup may come into contact with the composition contained within the aluminium can body, particularly when the can is inverted. The mounting cup typically has a valve present in a hole in its centre. In use, opening of the valve allows the antiperspirant aerosol composition to leave the aluminium can body via a nozzle and thereby produce a spray.

The mounting cup may be lacquered on its external surface; however, it is its internal surface that is more frequently in contact with the composition and it is this side that must be protected by an EP-vinyl lacquer. From the preceding sentence, it will be understood that the bottom side of the mounting cup is its internal surface when the mounting cup is in place on the aluminium can body. The EP-vinyl lacquer is a layer of material applied to the internal surface of the mounting cup by methods known in the art. The layer is preferably of thickness from 1 to 500 microns at all points on the internal surface of the can, the high maximum being due to the difficulty of applying lacquer to the mounting cup because of its highly contoured nature. The average thickness of the layer is preferably from 1 to 50 microns and may be measured by making 10 or more measurements at random positions on the bottom side of the mounting cup.

The EP-vinyl lacquer, otherwise known as organosol or Micoflex lacquer, is actually a dispersion of very finely powdered PVC [poly(vinyl chloride)] in an epoxy-phenolic (EP) matrix. It is well known in the art and has been commercially available for many years.

PAM lacquer was found to be unsuitable for the internal surface of the mounting cup because of the fact that this lacquer is particularly difficult to apply and the mounting cup is highly contoured, making its application to that component especially difficult.

A mounting cup provided with EP-vinyl lacquer on its internal surface has been found to be particularly beneficial in terms of corrosion resistance, when combined with the other features of the present invention (vide infra). For this reason, EP-vinyl lacquer is the lacquer of choice for the internal surface of the mounting cup, despite the fact this lacquer is particularly expensive. The expensive nature of EP-vinyl lacquer, and the presence of PVC as one of its components, are reasons for its non-use on the larger area of the internal surface of the aluminium can.

In order to further enhance corrosion resistance, it is preferred that the mounting cup, like the can body, is made from aluminium. For the same reason, it also preferred that the mounting cup is lacquered on its external surface with EP-vinyl lacquer, as well as on its internal surface.

### Examples

In the following examples, comparative examples are indicated by letters and examples according to the invention are indicated by numbers. All amounts are percentages by weight of the total composition.

The antiperspirant aerosol composition indicated in Table 1 was prepared as a 'dual emulsion' by a method analogous to that used to prepare the similar examples detailed in WO 03/105795 (Brown et al).

**Table 1: Antiperspirant Aerosol Composition**

| **Component** | **Trade name and supplier** | **Amount** |
|---|---|---|
| Silicone oil | DC 245 ex Dow Corning | 4.18 |
| PPG-14 butylether | Fluid AP ex Union Carbide | 2.0 |
| Hexyl decanol | Eutanol G16 *ex* Henkel | 0.84 |
| Fragrance | | 1 |
| 50% ACH solution | Aloxicol L *ex* Guilini | 20 |
| Cetyl dimethicone copolyol | Abil EM 90 *ex* Goldschmidt | 0.21 |
| PVM/MA copolymer | Gantrez S90 *ex* ISP | 2.02 |
| Water | | 11.48 |
| Propellant | CAP 40 ex Color/BP | To 100 |

Samples of the composition detailed in Table 1 were filled into dispenser devices comprising the components indicated in the second and third columns of Table 2. It should be noted that 'valve cup' is an alternative term for 'mounting cup'. Table 2 also indicates the corrosion resistance of the various products.

**Table 2: Corrosion Test Results**

| **Example** | **Metal/Lacquer¹** | | **Corrosion²** | |
|---|---|---|---|---|
| | **Can body** | **Valve cup** | **Can body** | **Valve cup** |
| A | Tinplate/EPON | Tinplate/EPON | After 48 wk³ | After 12 wk. |
| B | Al/EPON | Tinplate/EPON | After 48 wk. | After 12 wk. |
| C | Al/PAM⁴ | Tinplate/EPON | None | After 12 wk. |
| D | Al/EPON | Al/Micoflex⁵ | After 48 wk. | None |
| 1 | Al/PAM⁴ | Al/Micoflex⁵ | None | None |
| E⁶ | Al/PAM⁴ | Al/EPON | None ⁷ | After 24 wk. |

| | | | | |
|---|---|---|---|---|
| 1. Lacquer present on the internal surface on the can body and the internal surface on the valve cup. 2. Inspections made every 12 weeks. Products stored at 45°C. 3. Mottling of the lacquer observed after 12 weeks. 4. Lacquered cans obtained from Boxal Group. 5. Lacquered mounting cups obtained from Precision Valve Corp. 6. Slightly different emulsion composition used - essentially the same as Composition 2, detailed below. 7. No corrosion after 24 weeks. | | | | |

Table 2 indicates that comparative examples A to E all showed signs of corrosion within 48 weeks, corrosion in some instances being observed on the first inspection after only 12 weeks. Only the example according to the invention showed no corrosion over the entire period of the test (48 weeks).

The compositions detailed in Table 3 have also been prepared and placed in dispensing devices comprising an aluminium can body internally lacquered with PAM and a mounting cup lacquered on its internal surface with EP-vinyl lacquer. These products were placed on storage at 45°C in both upright and inverted positions and inspections were made every 12 weeks. For the products comprising compositions 2 and 3, 48 weeks of storage data are available and for the products comprising compositions 4 and 5, 36 weeks of storage data are available. With the products comprising compositions 3, 4, and 5, no corrosion was observed throughout the test. With the product comprising composition 2, no corrosion of the can was observed throughout the test and no corrosion of the valve cup was observed after 24 weeks. After 36 weeks, slight corrosion pitting of the valve cup was observed for the products stored in an inverted position. No corrosion of the valve cup (even after 48 weeks) was observed for the products stored in an upright position.

Compositions 2, 3, 4, and 5 were prepared in the following manner. The DC245, Finsolv TN, Abil EM90, and fragrance were stirred together. The Aloxicol L and additional water were mixed and slowly added to the oils and emulsfier, with moderate shear. In the preparation of Compositions 3 and 5, the HDK H30 was mixed into the composition using moderate shear, followed by high shear to give the final emulsion 'base'. The bases were transferred to aluminium aerosol cans and gassed with the CAP 40 using standard procedures.

**Table 3: Further Compositions**

| **Component** | **Trade name** | **Composition** | | | |
|---|---|---|---|---|---|
| | | **2** | **3** | **4** | **5** |
| Silicone oil | DC 245 | 13.86 | 13.46 | 8.76 | 8.41 |
| Propellant | CAP 40 | 50 | 50 | 65 | 65 |
| C₁₂₋₁₅ alkyl benzoate | Finsolv TN | 2 | 2 | 2 | 2 |
| 50% ACH solution | Aloxicol L | 20 | 20 | 20 | 20 |
| Cetyl dimethicone copolyol | Abil EM 90 | 0.2 | 0.25 | 0.3 | 0.3 |
| Hydrophobically-modified silica | HDK H30, ex Wacker | -- | 0.35 | -- | 0.35 |
| Fragrance | | 0.6 | 0.6 | 0.6 | 0.6 |
| Water | | to 100 | to 100 | to 100 | to 100 |

## Claims

1. An antiperspirant aerosol product comprising an aqueous solution of antiperspirant active emulsified in an oily continuous phase and packaged in a dispensing device comprising an aluminium can body internally lacquered with polyamide imide resin and a mounting cup lacquered on its internal surface with epoxy-phenolic vinyl lacquer.

2. An antiperspirant aerosol product according to claim 1, wherein the mounting cup is made of aluminium.

3. An Antiperspirant aerosol product according to claim 1 or 2, wherein the mounting cup is lacquered on its external surface with epoxy-phenolic vinyl lacquer.

4. An antiperspirant aerosol product according to any of the preceding claims, comprising an antiperspirant aerosol composition comprising a volatile propellant.

5. An antiperspirant aerosol product according to claim 4, wherein the volatile propellant is present in an amount of from 30 to 65% by weight of the total composition.

6. An antiperspirant aerosol product according to claim 5, wherein the volatile propellant is present in an amount of from 30 to 50% by weight of the total composition.

7. An antiperspirant aerosol product according to any of the preceding claims, comprising an antiperspirant aerosol composition wherein the proportion of disperse phase is from 50% to 90% by weight of the composition, excluding any volatile propellant that may be present.

8. An antiperspirant aerosol product according to any of the preceding claims, comprising a silicone oil in the oily continuous phase and using an emulsifier that is a silicone derivative.

9. An antiperspirant aerosol product according to claim 8, comprising cetyl dimethicone copolyol or lauryl dimethicone copolyol.

10. An antiperspirant aerosol product according to any of the preceding claims, comprising from 10% to 30% by weight of antiperspirant active.

11. An antiperspirant aerosol product according to any of the preceding claims, comprising aluminium chlorohydrate antiperspirant active.

12. An antiperspirant aerosol product according to any of the preceding claims, wherein at least 90% of the droplets of aqueous phase dispersed in the oily continuous phase have a particle size of from 1 to 25 µm.

13. An antiperspirant aerosol product according to any of the preceding claims, wherein the oily continuous phase comprises from 20% to 40% by weight of the composition excluding any volatile propellant that may be present.

14. An antiperspirant aerosol product according to claim 13, wherein the oily continuous phase comprises from 25% to 35% by weight of the composition excluding any volatile propellant that may be present.

15. An antiperspirant aerosol product according to any of the preceding claims, comprising a proportion of emulsifier that is from 0.05% to 5% by weight of the composition.

16. An antiperspirant aerosol product according to claim 15, comprising a proportion of emulsifier that is from 0.1% to 2.5% by weight of the composition.

17. An antiperspirant aerosol product according to any of the preceding claims, comprising from 0.1% to 5% by weight of the composition of emulsifiers that are silicone derivatives.

18. An antiperspirant aerosol product according to claim 17, comprising from 0.25% to 0.4% by weight of the composition of emulsifiers that are silicone derivatives.

19. An antiperspirant aerosol product according to any of the preceding claims, wherein the weight ratio of volatile propellant to water is from 1.5:1 to 2.25:1.

20. An antiperspirant aerosol product according to any of the preceding claims, comprising an emollient oil that is an alkyl ester, benzoate ester, hydrogenated polyalkene, PPG ether, isopropyl palmitate, phenylsilicone, or isopropyl myristate.

21. An antiperspirant aerosol product according to any of the preceding claims, comprising cetyl dimethicone copolyol and an aqueous solution of antiperspirant active emulsified in an oily continuous phase comprising a silicone oil and an emollient oil that is an alkyl ester, benzoate ester, hydrogenated polyalkene, PPG ether, isopropyl palmitate, phenylsilicone, or isopropyl myristate.

22. An antiperspirant aerosol product according to any of the preceding claims, comprising cetyl dimethicone copolyol or lauryl dimethicone copolyol at from 0.1% to 5% by weight of the composition.

23. An antiperspirant aerosol product according claim 22, comprising cetyl dimethicone copolyol at from 0.1% to 5% by weight of the composition.

24. An antiperspirant aerosol composition according to any one of the preceding claims, comprising from 0.1% to 5% by weight of cetyl dimethicone copolyol, from 30% to 50% by weight of an aqueous solution of antiperspirant active emulsified in an oily continuous phase comprising a silicone oil and an emollient oil that is an alkyl ester, benzoate ester, hydrogenated polyalkene, PPG ether, isopropyl palmitate, phenylsilicone, or isopropyl myristate, the oily continuous phase comprising from 25% to 35% by weight of the composition excluding the volatile propellant and the weight ratio of volatile propellant to water being from 1.5:1 to 2.25:1, said composition being packaged in a dispensing device comprising an aluminium can body internally lacquered with polyamide imide resin and a mounting cup lacquered on its internal surface with epoxy-phenolic vinyl lacquer.

25. A method of dispensing an antiperspirant aerosol composition comprising an aqueous solution of antiperspirant active emulsified in an oily continuous phase, said method comprising:
i. the containment of said antiperspirant aerosol composition in a pressurised state in a dispensing device comprising an aluminium can body internally lacquered with polyamide imide resin and closed by a mounting cup lacquered on its internal surface with epoxy-phenolic vinyl lacquer;
ii. releasing said pressurised antiperspirant aerosol composition by the opening a valve located in the middle of the mounting cup; and
iii. passing the antiperspirant aerosol composition through a nozzle to produce a spray.

26. A method of manufacture of an antiperspirant aerosol product comprising the steps of:
i. dissolving an antiperspirant active in water;
ii. emulsifying the resulting aqueous solution of antiperspirant active in an oily continuous phase; packaging of resulting the oil-in-water emulsion composition in a dispensing device comprising an aluminium can body internally lacquered with polyamide imide resin and a mounting cup lacquered on its internal surface with epoxy-phenolic vinyl lacquer.

## Patentansprüche

1. Schweißhemmendes Aerosol-Produkt, umfassend eine wässrige Lösung eines schweißhemmenden aktiven Mittels, emulgiert in einer öligen kontinuierlichen Phase, und verpackt in einer Abgabevorrichtung, umfassend ein Dosengehäuse aus Aluminium, das innen mit einem Polyamidimidharz lackiert ist, und eine Montagekappe, die an ihrer Innenseite mit einem epoxyphenolischen Vinyllack lackiert ist.

2. Schweißhemmendes Aerosol-Produkt nach Anspruch 1, wobei die Montagekappe aus Aluminium besteht.

3. Schweißhemmendes Aerosol-Produkt nach Anspruch 1 oder 2, wobei die Montagekappe auf ihrer Außenseite mit einem epoxyphenolischen Vinyllack lackiert ist.

4. Schweißhemmendes Aerosol-Produkt nach einem der vorangehenden Ansprüche, umfassend eine schweißhemmende Aerosol-Zusammensetzung, die ein flüchtiges Treibmittel umfasst.

5. Schweißhemmendes Aerosol-Produkt nach Anspruch 4, wobei das flüchtige Treibmittel in einer Menge von 30 bis 65 Gewichts-% der gesamten Zusammensetzung vorliegt.

6. Schweißhemmendes Aerosol-Produkt nach Anspruch 5, wobei das flüchtige Treibmittel in einer Menge von 30 bis 50 Gewichts-% der gesamten Zusammensetzung vorliegt.

7. Schweißhemmendes Aerosol-Produkt nach einem der vorangehenden Ansprüche, umfassend eine schweißhemmende Aeorol-Zusammensetzung, in der der Verhältnisanteil von disperser Phase 50 bis 90 Gewichts-% der Zusammensetzung, ausschließlich eines flüchtigen Treibmittels, das vorliegen kann, ist.

8. Schweißhemmendes Aerosol-Produkt nach einem der vorangehenden Ansprüche, das ein Siliconöl in der öligen kontinuierlichen Phase umfasst und einen Emulgator verwendet, der ein Siliconderivat ist.

9. Schweißhemmendes Aerosol-Produkt nach Anspruch 8, das Cetyldimethiconcopolyol oder Lauryldimethiconcopolyol umfasst.

10. Schweißhemmendes Aerosol-Produkt nach einem der vorangehenden Ansprüche, das 10 Gewichts-% bis 30 Gewichts-% schweißhemmendes aktives Mittel umfasst.

11. Schweißhemmendes Aerosol-Produkt nach einem der vorangehenden Ansprüche, das Aluminiumchlorhydrat als schweißhemmendes aktives Mittel umfasst.

12. Schweißhemmendes Aerosol-Produkt nach einem der vorangehenden Ansprüche, wobei wenigstens 90 % der Tröpfchen der wässrigen Phase, dispergiert in der öligen kontinuierlichen Phase, eine Partikelgröße von 1 bis 25 µm haben.

13. Schweißhemmendes Aerosol-Produkt nach einem der vorangehenden Ansprüche, wobei die ölige kontinuierliche Phase 20 Gewichts-% bis 40 Gewichts-% der Zusammensetzung, ausschließlich eines flüchtigen Treibmittels, das vorliegen kann, ausmacht.

14. Schweißhemmendes Aerosol-Produkt nach Anspruch 13, wobei die ölige kontinuierliche Phase 25 Gewichts-% bis 35 Gewichts-% der Zusammensetzung, ausschließlich eines flüchtigen Treibmittels, das vorliegen kann, ausmacht.

15. Schweißhemmendes Aerosol-Produkt nach einem der vorangehenden Ansprüche, das einen Anteil an Emulgator umfasst, der 0,05 Gewichts-% bis 5 Gewichts-% ist.

16. Schweißhemmendes Aerosol-Produkt nach Anspruch 15, das einen Anteil an Emulgator umfasst, der 0,1 Gewichts-% bis 2,5 Gewichts-% der Zusammensetzung ist.

17. Schweißhemmendes Aerosol-Produkt nach einem der vorangehenden Ansprüche, das 0,1 Gewichts-% bis 5 Gewichts-% der Zusammensetzung an Emulgatoren umfasst, die Siliconderivate sind.

18. Schweißhemmendes Aerosol-Produkt nach Anspruch 17, das 0,25 Gewichts-% bis 0,4 Gewichts-% der Zusammensetzung an Emulgatoren umfasst, die Siliconderivate sind.

19. Schweißhemmendes Aerosol-Produkt nach einem der vorangehenden Ansprüche, wobei das Gewichtsverhältnis von flüchtigem Treibmittel zu Wasser von 1,5:1 bis 2,25:1 ist.

20. Schweißhemmendes Aerosol-Produkt nach einem der vorangehenden Ansprüche, umfassend ein Haut erweichendes Öl, das ein Alkylester, Benzoatester, hydriertes Polyalken, PPG-Ether, Isopropylpalmitat, Phenylsilicon oder Isopropylmyristat ist.

21. Schweißhemmendes Aerosol-Produkt nach einem der vorangehenden Ansprüche, umfassend Cetyldimethiconcopolyol und eine wässrige Lösung von schweißhemmendem aktivem Mittel, emulgiert in einer öligen kontinuierlichen Phase, und ein Haut erweichendes Öl, das ein Alkylester, Benzoatester, hydriertes Polyalken, PPG-Ether, Isopropylpalmitat, Phenylsilicon oder Isopropylmyristat ist.

22. Schweißhemmendes Aerosol-Produkt nach einem der vorangehenden Ansprüche, das Cetyldimethiconcopolyol oder Lauryldimethiconcopolyol mit 0,1 Gewichts-% bis 5 Gewichts-% der Zusammensetzung umfasst.

23. Schweißhemmendes Aerosol-Produkt nach Anspruch 22, das CetyldimethiconCopolyol mit 0,1 Gewichts-% bis 5 Gewichts-% der Zusammensetzung umfasst.

24. Schweißhemmende Aerosol-Zusammensetzung nach einem der vorangehenden Ansprüche, umfassend 0,1 Gewichts-% bis 5 Gewichts-% Cetyldimethiconcopolyol, 30 Gewichts-% bis 50 Gewichts-% einer wässrigen Lösung von schweißhemmendem aktivem Mittel, emulgiert in einer öligen kontinuierlichen Phase, umfassend ein Siliconöl und ein Haut erweichendes Mittel, das ein Alkylester, Benzoatester, hydriertes Polyalken, PPG-Ether, Isopropylpalmitat, Phenylsilicon oder Isopropylmyristat ist, wobei die ölige kontinuierliche Phase 25 Gewichts-% bis 35 Gewichts-% der Zusammensetzung, ausschließlich des flüchtigen Treibmittels, ausmacht, wobei das Gewichtsverhältnis von flüchtigem Treibmittel zu Wasser von 1,5:1 bis 2,25:1 ist, wobei diese Zusammensetzung in einer Abgabevorrichtung verpackt ist, umfassend ein Dosengehäuse aus Aluminium, das innen mit einem Polyamidimidharz lackiert ist, und eine Montagekappe, die an ihrer Innenseite mit einem epoxyphenolischen Vinyllack lackiert ist.

25. Verfahren zum Abgeben einer schweißhemmenden Aerosol-Zusammensetzung, umfassend eine wässrige Lösung von schweißhemmendem aktivem Mittel, emulgiert in einer öligen kontinuierlichen Phase, wobei das Verfahren umfasst:
i. Halten der schweißhemmenden Aerosol-Zusammensetzung in einem druckbeaufschlagten Zustand in einer Abgabevorrichtung, umfassend ein Dosengehäuse aus Aluminium, das innen mit einem Polyamidimidlack lackiert ist, und mit einer Montagekappe, die an ihrer Innenseite mit einen epoxyphenolischen Vinyllack lackiert ist, verschlossen ist;
ii. Freisetzen der druckbeaufschlagten schweißhemmenden Aerosol-Zusammensetzung durch das Öffnen eines Ventils, das sich in der Mitte der Montagekappe befindet, und
iii. Strömen der schweißhemmenden Aerosol-Zusammensetzung durch eine Düse, um ein Spray zu produzieren.

26. Verfahren zur Herstellung eines schweißhemmenden Aerosol-Produkts, umfassend die Schritte:
i. Lösen eines schweißhemmenden aktiven Mittels in Wasser;
ii. Emulgieren der resultierenden wässrigen Lösung von schweißhemmendem aktivem Mittel in einer öligen kontinuierlichen Phase, Verpacken der resultierenden Öl-in-Wasser-Emulsion-Zusammensetzung in einer Abgabevorrichtung, umfassend ein Dosengehäuse aus Aluminium, das innen mit einem Polyamidimidharz lackiert ist, und eine Montagekappe, die an ihrer Innenseite mit einem epoxyphenolischen Vinyllack lackiert ist.

## Revendications

1. Produit aérosol antitranspirant comprenant une solution aqueuse de principe actif antitranspirant émulsifiée dans une phase continue huileuse et emballée dans un dispositif distributeur comprenant un corps de boîte en aluminium revêtu à l'intérieur d'une laque de résine de polyamide imide et une coupelle de valve revêtue d'une laque de vinylépoxy-phénolique sur sa surface interne.

2. Produit aérosol antitranspirant selon la revendication 1, dans lequel la coupelle de valve est réalisée en aluminium.

3. Produit aérosol antitranspirant selon la revendication 1 ou 2, dans lequel la coupelle de valve est revêtue d'une laque de vinylépoxy-phénolique sur sa surface externe.

4. Produit aérosol antitranspirant selon l'une quelconque des revendications précédentes, comprenant une composition aérosol antitranspirante comprenant un agent propulseur volatil.

5. Produit aérosol antitranspirant selon la revendication 4, dans lequel l'agent propulseur volatil est présent dans une quantité allant de 30 à 65 % en poids de la composition totale.

6. Produit aérosol antitranspirant selon la revendication 5, dans lequel l'agent propulseur volatil est présent dans une quantité allant de 30 à 50 % en poids de la composition totale.

7. Produit aérosol antitranspirant selon l'une quelconque des revendications précédentes, comprenant une composition aérosol antitranspirante dans laquelle la proportion de phase dispersée est de 50 % à 90 % en poids de la composition, excluant tout agent propulseur volatil qui peut être présent.

8. Produit aérosol antitranspirant selon l'une quelconque des revendications précédentes, comprenant une huile de silicone dans la phase continue huileuse et utilisant un émulsifiant qui est un dérivé de silicone.

9. Produit aérosol antitranspirant selon la revendication 8, comprenant du cétyldiméthicone copolyol ou du lauryldiméthicone copolyol.

10. Produit aérosol antitranspirant selon l'une quelconque des revendications précédentes, comprenant de 10 % à 30 % en poids de principe actif antitranspirant.

11. Produit aérosol antitranspirant selon l'une quelconque des revendications précédentes, comprenant le principe actif antitranspirant chlorhydrate d'aluminium.

12. Produit aérosol antitranspirant selon l'une quelconque des revendications précédentes, dans lequel au moins 90 % des gouttelettes de la phase aqueuse dispersée dans la phase continue huileuse ont une taille de particule de 1 à 25 µm.

13. Produit aérosol antitranspirant selon l'une quelconque des revendications précédentes, dans lequel la phase continue huileuse comprend de 20 % à 40 % en poids de la composition excluant tout agent propulseur volatil qui peut être présent.

14. Produit aérosol antitranspirant selon la revendication 13, dans lequel la phase continue huileuse comprend de 25 % à 35 % en poids de la composition excluant tout propulseur volatil qui peut être présent.

15. Produit aérosol antitranspirant selon l'une quelconque des revendications précédentes, comprenant une proportion d'émulsifiant qui est de 0,05 % à 5 % en poids de la composition.

16. Produit aérosol antitranspirant selon la revendication 15, comprenant une proportion d'émulsifiant qui est de 0,1 % à 2,5 % en poids de la composition.

17. Produit aérosol antitranspirant selon l'une quelconque des revendications précédentes, comprenant de 0,1 % à 5 % en poids de la composition d'émulsifiants qui sont des dérivés de silicone.

18. Produit aérosol antitranspirant selon la revendication 17, comprenant de 0,25 % à 0,4 % en poids de la composition d'émulsifiants qui sont des dérivés de silicone.

19. Produit aérosol antitranspirant selon l'une quelconque des revendications précédentes, dans lequel le rapport en poids entre agent propulseur volatil et eau est de 1,5 : 1 à 2,25 : 1.

20. Produit aérosol antitranspirant selon l'une quelconque des revendications précédentes, comprenant une huile émolliente qui est un ester d'alkyle, ester de benzoate, poly(alcène)hydrogéné, éther de PPG, palmitate d'isopropyle, phénylsilicone ou myristate d'isopropyle.

21. Produit aérosol antitranspirant selon l'une quelconque des revendications précédentes, comprenant du cétyldiméthicone copolyol et une solution aqueuse de principe actif antitranspirant émulsifié dans une phase continue huileuse comprenant une huile silicone et une huile émolliente qui est un ester d'alkyle, ester de benzoate, poly(alcène) hydrogéné, éther de PPG, palmitate d'isopropyle, phénylsilicone ou myristate d'isopropyle.

22. Produit aérosol antitranspirant selon l'une quelconque des revendications précédentes, comprenant du cétyldiméthicone copolyol ou lauryldiméthicone copolyol à hauteur de 0,1 % à 5 % en poids de la composition.

23. Produit aérosol antitranspirant selon la revendication 22, comprenant du cétyldiméthicone copolyol à hauteur de 0,1 % à 5 % en poids de la composition.

24. Composition aérosol antitranspirante selon l'une quelconque des revendications précédentes, comprenant de 0,1 % à 5 % en poids de cétyldiméthicone copolyol, de 30 % à 50 % en poids d'une solution aqueuse de principe actif antitranspirant émulsifiée dans une phase continue huileuse comprenant une huile silicone et une huile émolliente qui est un ester d'alkyle, ester de benzoate, poly(alcène) hydrogéné, éther de PPG, palmitate d'isopropyle, phénylsilicone ou myristate d'isopropyle, la phase continue huileuse comprenant de 25 % à 35 % en poids de la composition excluant l'agent propulseur volatil et le rapport en poids entre l'agent propulseur volatil et l'eau étant de 1,5 : 1 à 2,25 : 1, ladite composition étant emballée dans un dispositif distributeur comprenant un corps de boîte en aluminium revêtu à l'intérieur d'une laque de résine de polyamide imide et une coupelle de valve revêtue d'une laque de vinylépoxy-phénolique sur sa surface interne.

25. Procédé de distribution d'une composition aérosol antitranspirante comprenant une solution aqueuse de principe actif antitranspirant émulsifiée dans une phase continue huileuse, ledit procédé comprenant :
i. le confinement de ladite composition aérosol antitranspirante dans un état pressurisé dans un dispositif distributeur comprenant un corps de boîte en aluminium revêtu à l'intérieur d'une laque de résine de polyamide imide et fermé par une coupelle de valve revêtue d'une laque de vinylépoxy-phénolique sur sa surface interne ;
ii. la libération de ladite composition aérosol antitranspirante pressurisée par l'ouverture d'une valve située au milieu de la coupelle de valve ; et
iii.le passage de la composition aérosol antitranspirante à travers une buse pour produire une pulvérisation.

26. Procédé de fabrication d'un produit aérosol antitranspirant comprenant les étapes suivantes :
i. dissoudre un principe actif antitranspirant dans l'eau ;
ii. émulsifier la solution aqueuse résultante de principe actif antitranspirant dans une phase continue huileuse ;
emballer la composition d'émulsion huile dans l'eau résultante dans un dispositif distributeur comprenant un corps de boîte en aluminium revêtu à l'intérieur d'une laque de résine de polyamide imide et une coupelle de valve revêtue d'une laque de vinylépoxy-phénolique sur sa surface interne.
